Europäisches Patentamt

(19) European Patent Office

Office européen des brevets

(11) Numéro de publication: **0 268 646**

**B1**

(12) **FASCICULE DE BREVET EUROPEEN**

(45) Date de publication du fascicule du brevet: **03.10.90**

(51) Int. Cl.⁵: **G 01 N 31/12, G 01 N 33/28**

(21) Numéro de dépôt: **87903705.9**

(22) Date de dépôt: **04.06.87**

(86) Numéro de dépôt international:
**PCT/FR87/00201**

(87) Numéro de publication internationale:
**WO 87/07721 17.12.87 Gazette 87/28**

(54) **SYSTEME D'ANALYSE DU SOUFRE DANS LES HYDROCARBURES LIQUIDES.**

(30) Priorité: **05.06.86 FR 8608131**

(43) Date de publication de la demande:
**01.06.88 Bulletin 88/22**

(45) Mention de la délivrance du brevet:
**03.10.90 Bulletin 90/40**

(84) Etats contractants désignés:
**BE DE GB IT NL**

(56) Documents cités:
**FR-A-2 499 249
US-A-3 547 590
US-A-4 569 918**

(73) Titulaire: **ELF FRANCE
2 place de la Coupole La Défense 6
F-92400 Courbevoie (FR)**

(72) Inventeur: **FAURE, Dominique
21 Avenue Maréchal Foch
F-69110 Sainte-Foy-les-Lyon (FR)**
Inventeur: **SCRAMONCIN, Claude
60, rue Louis Braille
F-69800 Saint Priest (FR)**
Inventeur: **DEROUX, Gilbert
28, chemin de Torgne
F-38200 Serpaize (FR)**

(74) Mandataire: **Boillot, Marc
SOCIETE NATIONALE ELF AQUITAINE Division
Propriété Industrielle Tour Elf
F-92078 Paris la Défense Cédex 45 (FR)**

Courier Press, Leamington Spa, England.

## Description

L'invention a pour objet un système d'analyse du soufre dans les hydrocarbures liquides comportant principalement un four pour la combustion de l'échantillon et la transformation du soufre en $SO_2$, une cellule de mesure du $SO_2$ par fluorescence UV et un ensemble de traitement du signal.

Elle a également pour objet un procédé d'analyse du soufre utilisant ce système.

Des systèmes de ce type sont déjà connus, notamment celui décrit dans le brevet des Etats-Unis no 4.077.774 avec lequel on peut analyser le dioxyde de soufre, polluant de l'atmosphère, tout en éliminant par combustion les hydrocarbures éventuellement présents dans celle-ci.

On connaît également, d'après le brevet français no 2.499.249 un dispositif d'analyse du soufre qui comprend au moins un four de minéralisation, un four de combustion et un analyseur-détecteur de $SO_2$ par fluorescence UV suivis en aval, d'un dispositif de complément de débit et d'une pompe aspirante à débit et pression stabilisés destinée à l'évacuation des résidus de l'analyse.

On connaît encore d'après le brevet US 3.547.590 un dispositif d'analyse du soufre qui comprend une boucle de circulation d'échantillon, une vanne d'injection, des conduites d'alimentation en oxygène et gaz vecteur, une pompe d'aspiration de l'échantillon, et une électrovanne dans la boucle de circulation.

Bien que fonctionnant de façon satisfaisante, les systèmes décrits par ce brevet ne sont utilisables qu'en laboratoire et ne permettent pas l'analyse en ligne des hydrocarbures contenant du soufre et circulant dans une installation de fabrication industrielle.

Le but de l'invention est de créer un analyseur du soufre dans les hydrocarbures qui soit utilisable en ligne, fonctionne de façon pratiquement autonome, et permette éventuellement l'automatisation de l'installation de fabrication sur laquelle on l'utilise.

A cet effet, l'invention a pour objet un analyseur en ligne du soufre total dans les hydrocarbures liquides comportant principalement un four pour la combustion de l'échantillon et la transformation du soufre en $SO_2$, une cellule de mesure du $SO_2$ par fluorescence UV et un ensemble électronique de traitement du signal et de commande de l'analyseur, comportant également une boucle primaire de circulation d'échantillon fermée sur une installation à contrôler, une boucle secondaire de circulation d'échantillon, une vanne d'injection à six voies, ainsi qu'un réservoir d'échantillon étalon et deux conduites d'alimentation en oxygène et en gaz vecteur. La boucle secondaire part de la boucle primaire et passe ensuite par un filtre, une pompe, une électrovanne simple et ladite vanne à six voies et se referme ensuite sur la boucle primaire, le réservoir d'échantillon étalon est raccordé à la boucle secondaire par l'intermédiaire d'une conduite dans laquelle se trouve une pompe et une électrovanne, une conduite d'alimentation en gaz vecteur est branchée sur la vanne à six voies et l'autre conduite d'alimentation en oxygène sur le four pour assurer respectivement le transport de la prise d'essai et sa combustion dans le four. La cellule de mesure est traversée par les gaz de combustion, à débit constant et l'excédent est évacué par une conduite vers l'extérieur.

Selon une autre caractéristique de l'invention, l'ensemble électronique assure:

la commande de la régulation du four, et des séquences d'étalonnage et d'analyse,

le déclenchement et l'identification des alarmes,

le traitement du signal, notamment le calcul et la transmission des résultats,

éventuellement, la transmission d'informations pour la commande directe de l'installation à contrôler.

L'invention a encore pour objet un procédé d'analyse de soufre dans les hydrocarbures dans lequel on oxyde les hydrocarbures dans un four, on fait passer les gaz de combustion dans une cellule de fluorescence UV et on intègre le signal de sortie de la cellule, l'intégrale de la courbe de ce signal étant alors proportionnelle à la teneur en soufre total dans ces hydrocarbures, on prélève les hydrocarbures à analyser directement sur une installation industrielle à l'aide d'une boucle primaire, on prélève une partie de ces hydrocarbures dans cette boucle primaire à l'aide d'une boucle secondaire, on fait passer la totalité de ce débit secondaire par la boucle de prise d'essai d'une vanne d'injection à six voies deux positions que l'on actionne périodiquement en vue de l'entraînement de la prise d'essai par le gaz vecteur et, entre ces injections périodiques d'échantillon, on intercale des injections d'hydrocarbure d'étalonnage substitué dans la boucle secondaire à l'hydrocarbure à analyser par un jeu de vannes qui interrompt et rétablit la circulation de ces hydrocarbures en fonction du programme inscrit dans l'ensemble électronique.

Selon encore une autre caractéristique de l'invention, on utilise un hydrocarbure d'étalonnage de composition voisine de celle de l'échantillon à analyser et on fixe des valeurs de consigne telles que le nombre d'injections consécutives d'hydrocarbure d'étalonnage et le nombre d'injections consécutives d'hydrocarbures à analyser, la durée d'une analyse qui est la durée qui sépare par exemple le début de deux injections consécutives.

Selon encore une autre caractéristique de l'invention, on calcule la valeur de la surface des pics d'étalonnage consécutifs et on les valide deux à deux à l'intérieur d'un écart relatif autorisé et on procède ensuite à la même validation pour les séries d'échantillonage consécutives, une alarme étant déclenchée si l'écart autorisé est dépassé.

La nature de l'incident étant identifiée, on déclenche une alarme et/ou on met l'appareil en sécurité et/ou coupe certains circuits en cas de dépassement de certaines valeurs de consigne telles que, par exemple, les températures trop hautes ou trop basses du four, une surface de pic nulle ou insuffisante, une dérive non autorisée de la ligne de base, un défaut de débit d'oxygène.

Suivant une autre caractéristique de l'invention, il est encore avantageux que l'on déclenche une alarme non accompagnée d'un arrêt automatique d'une partie quelconque ni de l'ensemble de l'analyseur lorsque la teneur en soufre total franchit un valeur limite haute fixée par l'exploitant.

L'invention sera mieux comprise à l'aide de la description qui va suivre et des dessins joints dans lesquels:

la figure 1 est un schéma de principe de l'analyseur du soufre dans les hydrocarbures selon l'invention.

Les figures 2a, 2b, 2c représentent schématiquement le fonctionnement de la vanne d'injection à six voies,

la figure 3 représente le brûleur utilisé dans le four de l'analyseur selon l'invention, et

la figure 4 représente un enregistrement partiel d'une analyse effectuée à l'aide de l'analyseur selon l'invention.

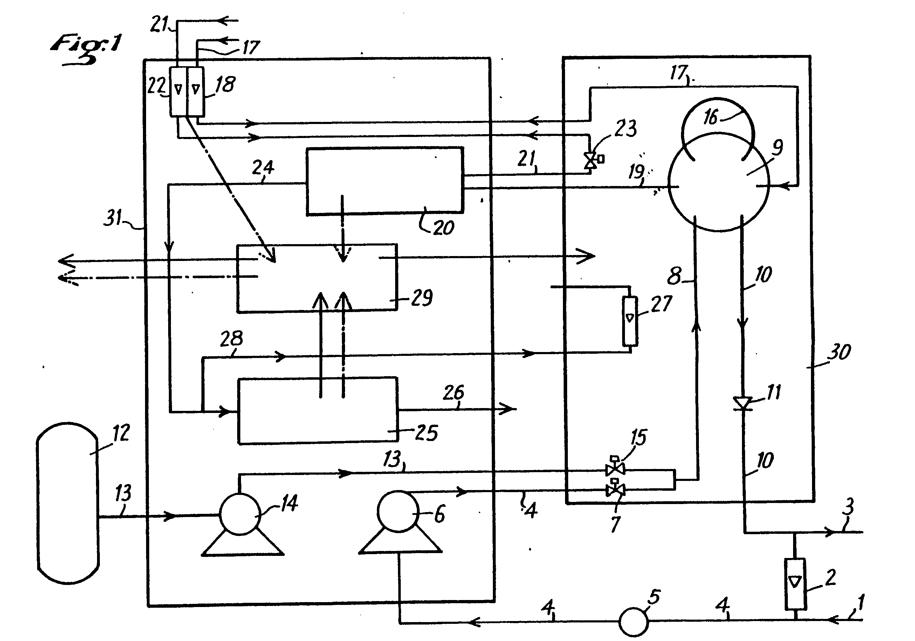

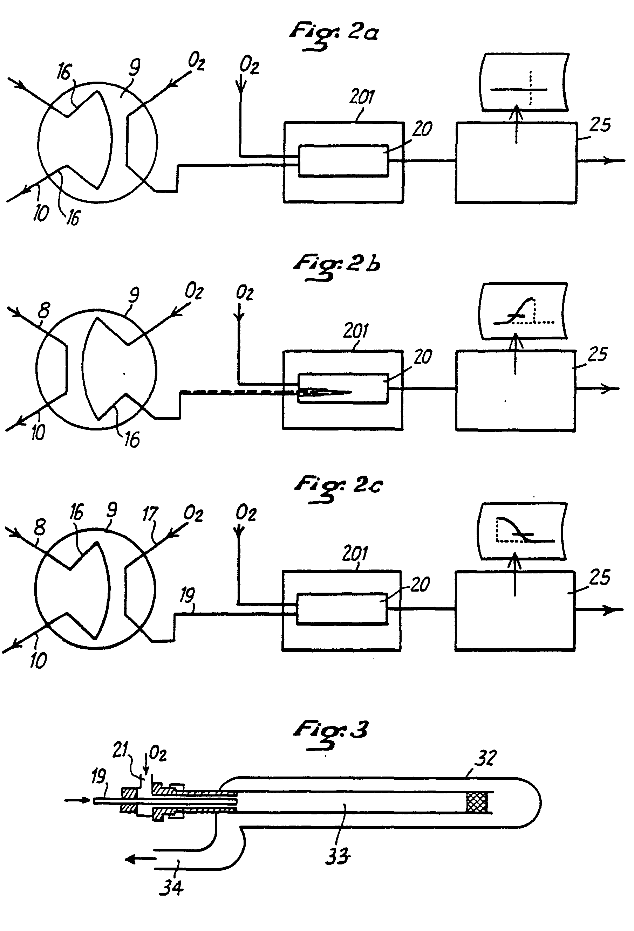

Sur la figure 1, le produit à analyser provient d'une installation industrielle (non représentée) et il y retourne de façon continue en passant par la conduite 1, le débitmètre 2 et la conduite 3 constituant ensemble la partie de la boucle primaire 1, 2, 3 sur laquelle on prélève l'échantillon. Ce dernier passe par la conduite 4, le filtre 5, la pompe 6, l'électrovanne tout ou rien 7, la conduite 8, la vanne d'injection à six voies (deux positions) 9, la boucle de prise d'essai 16, à nouveau la vanne 9 et la conduite 10 comportant le clapet antiretour 11 constituant ensemble une boucle secondaire 4—11, l'échantillon non utilisé retourne à l'installation par la conduite 3. La partie 8 à 11 de la boucle secondaire peut également être alimentée, après arrêt de la pompe 6 et fermeture de la vanne 7, en échantillon étalon contenu dans un réservoir 12 d'où on le prélève par la conduite 13 à l'aide de la pompe 14 et d'une électrovanne tout ou rien 15. Dans la vanne d'injection à six voies 9, l'échantillon circule dans une boucle de volume constant 16 (fig. 2a). Au changement de position de la vanne 9 (fig. 2b), cette boucle de volume constant 16 où est retenue la prise d'essai à analyser, est balayée par le gaz vecteur introduit par la conduite 17 et le débitmètre 18, la prise d'essai est alors transportée par la conduite 19 dans le four 20 isolé dans une enceinte 201. La vanne 9 est ensuite ramenée à sa position initiale (fig. 2c). Le four 20 est alimenté en permanence en oxygène de combustion par la conduite 21 comportant un débitmètre 22 et une électrovanne 23. L'effluent du four 20 est amené par la conduite 24 à la cellule 25 de mesure du SO$_2$ par fluorescence UV et il est ensuite évacué par la conduite 26. La cellule 25 peut être contournée par un bipasse 28 pour'viter qu'un débit trop important ne la parcoure. L'excès d'effluent gazeux est évacué directement par la conduite 28, un débitmètre 27 permettant à tout moment de vérifier l'existence de cet excès. Le rayonnement de fluorescence réémis dans la cellule 25 est transformé en signal traité par l'ensemble électronique 29. Cet ensemble électronique 29 comprend des éléments connus tels que par exemple un automatisme qui assure la commande des vannes et des pompes, la marche du four, la transmission des résultats et l'identification des alarmes selon un programme imposé.

Pour pouvoir être placé au voisinage de l'installation industrielle à contrôler et en respecter les consignes de sécurité, on peut regrouper les diverses parties de l'analyseur dans une armoire pressurisée 30 dont on a régulé la température et la pression et à laquelle sont adjoints des équipements externes ADF dont certains sont contenus dans un coffret 31.

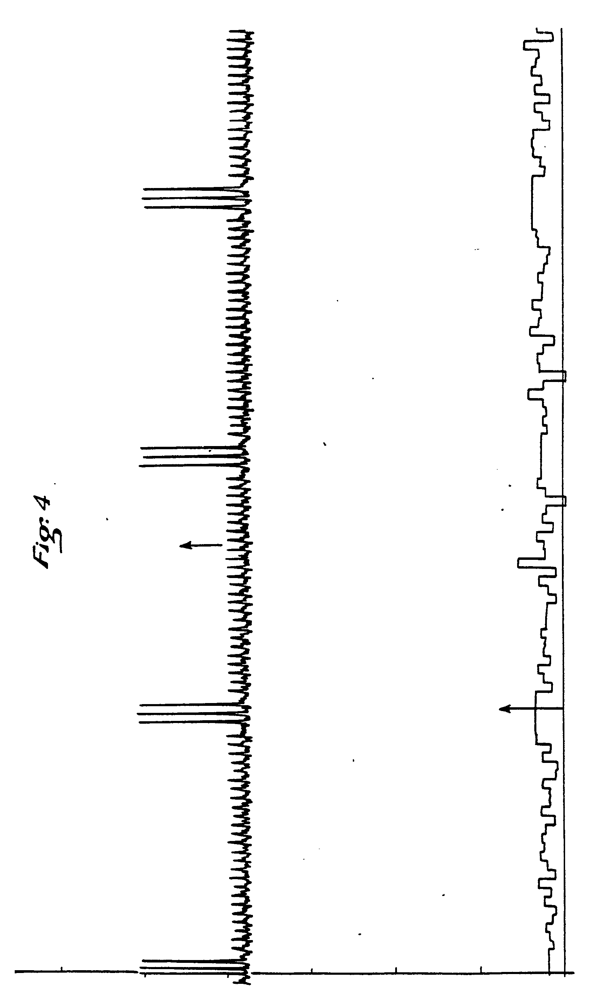

Sur la figure 3, un brûleur 32 est constitué d'une arrivée 19 de gaz vecteur entourée d'une arrivée concentrique 21 d'oxygène de combustion qui débouchent dans un tube commun 33. Les effluents de combustion repartent par le tube extérieur concentrique 34 à contre-courant de leur arrivée. La combustion de l'échantillon s'opère dans le four porté à 950°C à l'aide d'une résistance électrique (non représentée) régulée par l'ensemble électronique.

La figure 4 représente une fraction d'enregistrement du signal de mesure sur laquelle sont indiquées des séquences de trois déterminations consécutives de la teneur en soufre de l'étalon et vingt-cinq mesures consécutives sur l'échantillon à analyser.

Bien entendu, l'invention n'est pas limitée à l'exemple ci-dessus décrit et représenté à partir duquel l'homme de l'art pourra réaliser d'autres variantes et l'on peut, notamment dans le cas d'un échantillon d'hydrocarbure à trop forte teneur en soufre, procéder à la dilution de l'échantillon avant son analyse ou diluer le gaz de combustion par tout moyen convenable avant de le faire passer dans la cellule de fluorescence.

**Revendications**

1. Analyseur de la teneur en soufre total en ligne dans les hydrocarbures liquides comportant principalement un four pour la combustion de l'échantillon et la transformation du soufre en SO$_2$, une cellule de mesure du SO$_2$ et un ensemble électronique de traitement du signal et de commande de l'analyseur, et également une boucle de circulation d'échantillon (4—11), une vanne d'injection (9) et deux conduites (21) et (17) d'alimentation en oxygène et en gaz vecteur, la boucle de circulation d'échantillon (4—11) passant par un filtre (5), une pompe (6), une électrovanne simple (7) et la vanne (9), la conduite (17) étant branchée sur la vanne (9) et la conduite (19) sur le four (20) pour assurer, respectivement, le transport de la prise d'essai et sa combustion dans le four (20) caractérisé en ce qu'il comporte aussi une boucle primaire de circulation d'échantillon (1—3) fermée sur une installation à contrôler, ainsi qu'un réservoir d'échantillon étalon (12), et que la vanne d'injection est une vanne à 6 voies (9), la boucle de circulation d'échantillon (4—11) partant de la boucle primaire (1) et se refermant ensuite sur la boucle primaire (1—3), le réservoir (12) étant raccordé à la boucle de circulation de l'échantillon (4—11) par l'intermédiaire d'une conduite (13) dans laquelle se trouve une pompe (14) et une électrovanne (15), la cellule de mesure du SO$_2$ (25) qui utilise la fluorescence UV étant traversée, par les gaz de combustion, à débit constant et l'excédent étant évacué par la conduite (28) vers l'extérieur.

2. Analyseur de soufre total selon la revendication 1 caractérisé en ce que l'ensemble électronique assure:

la commande de la régulation du four, et des séquences d'étalonnage et d'analyse,

le déclenchement et l'identification des alarmes,

le traitement du signal, notamment le calcul et la transmission des résultats,

éventuellement, la transmission d'informations pour la commande directe de l'installation à contrôler.

3. Procédé d'analyse de la teneur en soufre total dans les hydrocarbures dans lequel on prélève les hydrocarbures à analyser directement sur une installation industrielle à l'aide d'une boucle de circulation d'échantillon, on injecte en discontinu à l'aide d'une vanne deux positions des volumes constants dans un four, on oxyde les hydrocarbures dans le four, on fait passer les gaz de combustion dans une cellule de mesure de SO$_2$ et on intègre le signal de sortie de la cellule, l'intégrale de ce signal étant alors proportionnelle à la teneur en soufre total dans ces hydrocarbures, caractérisé en ce que la vanne est actionnée périodiquement et que, entre ces injections périodiques d'échantillon, on intercale des injections d'hydrocarbure d'étalonnage substitué dans la boucle à l'hydrocarbure à analyser par un jeu de vannes qui interrompt et rétablit la circulation de ces hydrocarbures en fonction du programme inscrit dans l'ensemble électronique.

4. Procédé selon la revendication 3 caractérisé en ce que le nombre d'injections consécutives d'hydrocarbure d'étalonnage et le nombre d'injections consécutives d'hydrocarbures à analyser sont fixés comme valeurs de consigne.

5. Procédé selon la revendication 3 caractérisé en ce que la durée d'analyse est fixée comme valeur de consigne.

6. Procédé selon l'une quelconque des revendications 3 à 5 caractérisé en ce que l'on calcule la valeur de la surface des pics d'étalonnage consécutifs et on les valide deux à deux à l'intérieur d'un écart relatif autorisé et on procède ensuite à la même validation pour les séries d'étalonnages consécutives, une alarme étant déclenchée si l'écart autorisé est dépassé.

7. Procédé selon l'une quelconque des revendications 3 à 6 caractérisé en ce que, la nature de l'incident étant identifiée, on déclenche une alarme et/ou on met l'appareil en sécurité et/ou coupe certains circuits en cas de dépassement de valeurs de consigne prédéterminées.

8. Procédé selon l'une quelconque des revendications 3 à 7 caractérisé en ce que l'on déclenche une alarme non accompagnée d'un arrêt automatique d'une partie quelconque ni de l'ensemble de l'analyseur lorsque la teneur en soufre dépasse une valeur limite haute fixée par l'exploitant.

**Patentansprüche**

1. Prozeßgekoppelter Analysator für den Gesamtschwefelgehalt in flüssigen Kohlenwasserstoffen, welcher insbesondere einen Ofen für die Probenverbrennung und die Umwandlung des Schwefels in SO$_2$, eine Meßzelle für SO$_2$ und eine elektronische Anordnung für die Signalverarbeitung und die Analysatorsteuerung sowie einen probenumwälzkreislauf (4 bis 11), ein Einspritzventil (9) und zwei Leitungen (21) und (17) für die Zuführung von Sauerstoff und einem Trägergas aufweist, wobei der Probenumwälzkreislauf (4 bis 11) durch einen Filter (5), eine Pumpe (6), ein einfaches Magnetventil (7) und das Ventil (9) führt, wobei die Leitung (17) vom Ventil (9) und die Leitung (19) vom Ofen (20) abzweigt, um jeweils den Transport der Probe und ihre Verbrennung in dem Ofen (20) zu gewährleisten, dadurch gekennzeichnet, daß er auch einen primären Probenumwälzkreislauf (1 bis 3), der über eine zu steuernde Einrichtung geschlossen ist, sowie einen Eichprobenspeicher (12) aufweist, und daß das Einspritzventil ein Sechswegeventil (9) ist, wobei der Probenumwälzkreislauf (4 bis 11) von dem primären Kreislauf (1) ausgeht und sich dann auf dem primären Kreislauf (1 bis 3) wieder schließt, der Speicher (12) mit dem Probenumwälzkreislauf (4 bis 11) über eine Leitung (13) verbunden ist, in welcher sich eine Pumpe (14) und ein Magnetventil (15) befinden, die Meßzelle (15) für SO$_2$, die UV-Floureszenz verwendet, von den Verbrennungsgasen mit konstantem Durchsatz durchströmt wird und der Überschuß durch die Leitung (28) nach außen abgeführt wird.

2. Analysator für Gesamtschwefel nach Anspruch 1, dadurch gekennzeichnet, daß die elektronische Anordnung die Steuerung der Ofenregulierung und der Eich- und Analysesequenzen, das Auslösen und die Identifizierung von Alarmen, die Signalverarbeitung, insbesondere die Berechnung und die Übertragung von Ergebnissen und eventuell die Übertragung von Informationen für die direkte Steuerung der zu steuernden Einrichtung gewährleistet.

3. Verfahren zum Analysieren des Gesamtschwefelgehaltes in Kohlenwasserstoffen, bei welchem die zu analysierenden Kohlenwasserstoffe direkt einer industriellen Anlage mit Hilfe eines Probenumwälzkreislaufs entnommen werden, mit Hilfe eines Zweistellungsventils konstante Volumina diskontinuierlich in einen Ofen eingespritzt werden, mit Hilfe eines Zweistellungsventils konstante Volumina diskontinuierlich in einen Ofen eingespritzt werden, die Kohlenwasserstoffe in dem Ofen oxidiert werden, die Verbrennungsgase in eine Meßzelle für SO$_2$ geführt werden und das Ausgangssignal aus der Zelle integriert wird, wobei das Integral dieses Signals dann proportional zum Gesamtschwefelgehalt in diesen Kohlenwasserstoffen ist, dadurch gekennzeichnet, daß das Ventil periodisch betätigt wird, und daß zwischen diese periodischen Probeneinspritzungen Kohlenwasserstoffeinspritzungen zur Eichung zwischengeschaltet werden, die in dem Kreislauf an die Stelle des zu analysierenden Kohlenwasserstoffes durch einen Satz von Ventilen gesetzt ist, der die Umwälzung dieser Kohlenwasserstoffe als Funktion des in die elektronische Anordnung eingegebenen programms unterbricht und wiederherstellt.

4. Verfahren nach Anspruch 3, dadurch gekennzeichnet, daß die Anzahl der aufeinanderfolgenden Einspritzungen von Eichungskohlenwasserstoff und die Anzahl von aufeinanderfolgenden Einspritzungen von zu analysierenden Kohlenwasserstoffen als Einstellwerte festgelegt werden.

5. Verfahren nach Anspruch 3, dadurch gekennzeichnet, daß die Dauer der Analyse als Einstellwert festgelegt wird.

6. Verfahren nach einem der Ansprüche 3 bis 5, dadurch gekennzeichnet, daß der Flächenwert der aufeinanderfolgenden Eichmaxima berechnet wird, sie paarweise innerhalb einer zulässigen relativen Abweichung gültig gemacht werden, und daß dann die gleiche Gültigmachung für die darauffolgenden Reihen von Eichungen durchgeführt wird, wobei ein Alarm ausgelöst wird, wenn die zulässige Abweichung überschritten wird.

7. Verfahren nach einem der Ansprüche 3 bis 6, dadurch gekennzeichnet, daß bei identifizierter Art der Störung ein Alarm ausgelöst wird und/oder die Vorrichtung geschützt wird und/oder bestimmte Schaltungen abgetrennt werden im Falle der Überschreitung der vorher festgelegten Einstellwerte.

8. Verfahren nach einem der Ansprüche 3 bis 7, dadurch gekennzeichnet, daß ein Alarm ausgelöst wird, der nicht von einem automatischen Anhalten irgendeines Teils oder des gesamten Analysators begleitet ist, wenn der Schwefelgehalt einen hohen Grenzwert überschreitet, der vom Betreiber festgelegt ist.

**Claims**

1. Analyser for the on-line analysis of the total content of sulphur in liquid hydrocarbons, principally comprising a furnace for the combustion of the sample and transformation of the sulphur into $SO_2$, a $SO_2$ measuring cell and an electronic assembly for processing the signal and controlling the analyser, and likewise a sample circulating loop (4—11), an injection valve (9) and two oxygen and carrier gas supply pipes (21 and 17), the sample circulating loop (4—11) passing through a filter (5), a pump (6), a simple solenoid valve (7) and the valve (9), pipe (17) being connected to the valve (9) and the pipe (19) being connected to the furnace (20) in order to ensure respectively that the test sample is conveyed into and burnt in the furnace (20), characterised in that it also comprises a closed sample circulating loop (1—3) on an installation to be monitored, as well as a standard sample reservoir (12); and in that the injection valve is a six-way valve (9), the sample circulating loop (4—11) starting at the primary loop (1) and subsequently closing on the primary loop (1—3), the reservoir (12) being connected to the sample circulating loop (4—11) by means of a pipe (13) in which there is located a pump (14) and a solenoid valve (15), the combustion gases flowing through the $SO_2$ measuring cell (25), which uses UV fluorescence, at a constant rate and the excess being discharged by the pipe (28) towards the exterior.

2. Total sulphur analyser according to Claim 1, characterised in that the electronic assembly:

controls the furnace regulation and the standardisation and analysis sequences;

triggers and identifies the alarms;

processes the signal, in particular the calculation and transmission of the results;

if necessary, transmits the data for the direct control of the installation to be monitored.

3. Process for the analysis of the total sulphur content in hydrocarbons in which the hydrocarbons to be analysed are taken directly from an industrial plant by means of a sample circulating loop, constant volumes are injected discontinuously by means of a two-position valve into a furnace, the hydrocarbons are oxidised in the furnace, the combustion gases are passed into a $SO_2$ measuring cell and the cell output signal is integrated, the integral of this signal then being proportional to the total sulphur content in these hydrocarbons, characterised in that the valve is activated periodically and that there are interposed between these periodic sample injections, injections of substituted standardising hydrocarbons into the hydrocarbon loop to be analysed by a set of valves which interrupt and re-establish the circulation of the hydrocarbons according to the program function recorded in the electronic assembly.

4. Process according to Claim 3, characterised in that the number of consecutive standardising hydrocarbon injections and the number of consecutive hydrocarbon injections to be analysed are set as reference values.

5. Process according to Claim 3, characterised in that the duration of the analysis is set as the reference value.

6. Process according to any one of Claims 3 to 5, characterised in that the value of the surface area of the consecutive standardising peaks is calculated and they are validated in pairs within a permitted relative variation and the same validation process is subsequently performed for the series of consecutive standardising phases, an alarm being triggered if the permitted tolerance is exceeded.

7. Process according to any one of Claims 3 to 6, characterised in that once the nature of the incident has been identified, an alarm is triggered and/or the apparatus is made safe and/or certain circuits are broken if the predetermined reference values are exceeded.

8. Process according to any one of Claims 3 to 7, characterised in that an alarm is triggered which is not accompanied by an automatic stoppage neither of any part of, nor the entire, analyser when the sulphur content exceeds a top limit value fixed by the user.

Fig.1

Fig. 2a

Fig. 2b

Fig. 2c

Fig. 3

Fig. 4